# EUROPEAN PATENT APPLICATION

(11) **EP 0 533 938 A1**
(43) Date of publication of application: **31.03.1993**
(21) Application number: 92905332.0
(22) Date of filing: 19.02.1992
(51) Int. Cl.: A61K 37/02, A61K 47/12, A61K 47/16, A61K 47/42

(54) **COMPOSITION FOR RECTAL ADMINISTRATION OF DIFFICULTLY ABSORBABLE PEPTIDE**

(30) Priority: 19.02.1991 JP 45391/91
(71) Applicant: TSUMURA & CO., Chuo-ku, Tokyo 103 (JP)
(72) Inventor: AZECHI, Yasutaka, Ami-machi Inashiki-gun Ibaraki 300-11 (JP); KIMURA, Takayoshi, Ami-machi Inashiki-gun Ibaraki 300-11 (JP); YUN, Arifuku, Ami-machi Inashiki-gun Ibaraki 300-11 (JP)
(74) Representative: Wächtershäuser, Günter, Prof. Dr.
(86) International application number: JP9200168
(87) International publication number: WO9214479

(57) **Abstract**

A composition for the rectal administration of difficultly absorbable peptides, comprising a difficultly absorbable peptide as the active ingredient, a C₈ to C₁₄ saturated fatty acid or an avirulent salt thereof, and a protease inhibitor. The composition allows peptides which have been believed to be difficult to absorb to be absorbed efficiently through the rectal mucosa, so that it can achieve an excellent pharmacological efficacy without resort to injection which is painful and is limited in its administration. Further it has an economic advantage because it can provide a pharmaceutical preparation of relatively expensive peptides witha reduced loss thereof.

## Description

### Technical Field

The present invention relates to rectal preparations of low absorption peptides, and more specifically to rectal preparations for permitting rectal absorption of peptides which are susceptible to decomposition and therefore have low level of absorption.

### Background Art

For many years, administration of peptides having pharmacological activities are known to involve many problems. For example, even upon oral administration of a peptide having relatively good absorption through the digestive tube, its decomposition by peptide-digesting enzymes in the digestive tube cannot be avoided, thereby making it necessary to administer the peptide at a high dosage. This has posed a problem from the standpoint of economy.

On the other hand, peptides whose absorption in the digestive tube is difficult are generally administered by injection. However, injection is accompanied by painful discomfort and is not an administration route permitting easy practice. Injection is also accompanied by the problem that it involves potential dangers such as muscle contracture.

Under these circumstances, attempts have been made with a view toward administering physiologically-active peptides through the rectum. However, there are many peptides which cannot be absorbed through the rectum when administered as are, resulting in the outstanding desire for the development of a solution for this problem.

### Disclosure of the Invention

The present inventors have proceeded with extensive research to permit efficient absorption of a low absorption peptide through the rectal mucosa. As a result, it has been found that the above object can be achieved by the addition of the low absorption peptide to a base which contains a specific fatty acid or a non-toxic salt thereof and a protease inhibitor, leading to the completion of the present invention.

Namely, the present invention provides a rectal preparation of a low absorption peptide, which comprises the low absorption peptide as an effective ingredient, a saturated fatty acid having 8-14 carbon atoms or a non-toxic salt thereof, and a protease inhibitor.

### Brief Description of the Drawings

FIGURE 1 is a diagram showing variations of the calcitonin level in serum; and
FIGURE 2 through FIGURE 4 are diagrams illustrating variations of the calcium level in serum.

### Best Mode for Carrying Out the Invention

The term "a low absorption peptide" as the effective ingredient in the preparation according to the present invention (hereinafter referred to as the "peptide") means a peptide which is promptly decomposed in the mucosal endepidermis or the mucosal tissue and fails to exhibit good absorption. Examples of the peptide include those having a molecular weight greater than 1,500 and containing more than 10 amino acids, such as calcitonin and insulin.

The saturated fatty acid having 8-14 carbon atoms (hereinafter referred to as the "saturated fatty acid"), which is employed in the rectal preparation according to the present invention may be either one available from a natural substance or one obtained by chemical synthesis, with the former one being preferred. Examples of such saturated fatty acids include both straight fatty acids and branched fatty acids, with straight ones being preferred. The preferred carbon number of the saturated fatty acid can range from 10-12 (for example, caprylic acid or lauric acid), especially 10 (caprylic acid).

The non-toxic salt of the saturated fatty acid may be any salt insofar as it is pharmacologically acceptable. Examples include alkali metal salts such as the sodium and potassium salts and organic bases, e.g., basic amino acid salts such as the arginine and lysine salts.

Specific examples of the saturated fatty acid and its non-toxic salt include caprylic acid and its sodium, potassium, lysine and arginine salts; pelargonic acid and its sodium, potassium, lysine and arginine salts; capric acid and its sodium, potassium, lysine and arginine salts; undecylic acid and its arginine salt; lauric acid and its sodium, potassium, lysine and arginine salts; dodecylic acid and its lysine salt; myristic acid and its sodium, potassium, lysine and arginine salts. Among these, caprylic acids and its salts and lauric acid and its salts are particularly preferred.

These saturated fatty acids and their non-toxic salts can be used either singly or in combination. The saturated fatty acid or its non-toxic salt is generally added in a proportion of 0.1-20 wt.% (hereinafter merely indicated be "%"), preferably 1-15%, most preferably 3-10% based on the whole preparation.

Further, examples of the protease inhibitor employed in the present invention include gabexate mesylate (FOY), aprotinin, nafamostat mesylate, camostat mesylate, urinastatin, ε-aminocaproic acid, tranexamic acid and bestatin. The protease inhibitor is generally added in a proportion of 0.1-5% or so based on the whole preparation.

The rectal preparation according to the present invention can be obtained by methods known *per se* in the art, namely, by mixing and homogenizing the peptide as the effective ingredient, the saturated fatty acid and the protease inhibitor and then formulating the resulting mixture into a rectal preparation form. As one example of a preferred formulation process, the peptide and the saturated fatty acid are added to a melted base for rectal administration, followed by mixing. The protease inhibitor is added further and, after vigorous agitation, the resultant mixture is degasified. By methods known *per se* in the art, the mixture is poured in suitable amounts in plastic-made containers and is then solidified in a refrigerator. As an alternative process, the saturated fatty acid is dissolved in water and, if necessary, the resulting solution is isotonized and/or thickened. The peptide and the protease inhibitor are mixed under stirring to provide a rectal solution.

Upon formulation of the rectal preparation of this invention, various optional ingredients can also be incorporated as needed in addition to the three ingredients described above, including surfactants (sorbitan esters, sodium docusate, polyoxyethylene alkyl ethers, polyoxyethylene stearate, etc.); lubricants (sodium chloride, carnauba wax, silicon dioxide, zinc stearate, magnesium stearate, talc, etc.); solubilizing agents (cholesterol, stearic acid, sorbitan sodium docusate, triethanolamine, emulsified wax, etc.); antioxidants (ascorbic acid, citric acid, butylhydroxyanisole, butylhydroxytoluene, sodium metabisulfite, etc.); gloss agents (carnauba wax, silicon dioxide, zinc stearate, magnesium stearate, talc, etc.); coating agents (ethylcellulose, carnauba wax, carboxymethylcellulose sodium, shellac, cetyl alcohol, hydroxypropylcellulose, etc.); colorants (erythrosine, yellow orange S, indigotin, lake, iron oxide, β-carotene, caramel, etc.), pH adjustors (aminoacetic acid, ethanolamine, sodium chloride, carboxymethylcellulose, sodium carbonate, citric acid, etc.); solidifiers (bees wax, hydrogenated castor oil, cetyl ester wax, emulsified wax, paraffin, etc.); softening agents (starch, isopropyl myristate, glycerin monostearate, vaseline, lanolin alcohol, etc.); dispersants (polyoxyethylene castor oil derivatives, lecithin, etc.); solubilizers (polyoxyethylene castor oil derivatives, poloxamer, etc.); solubilizing aids (polyoxyethylene castor oil derivatives, poloxamer, etc.); antiseptics (glycerin, chlorohexidine hydrochloride, etc.); moistureproofing agents (calcium sulfate, etc.); preservatives (sodium benzoate, ethylparaben, benzalkonium chloride, chlorohexidine hydrochloride, chlorocresol, chlorobutanol, sorbic acid, butylparaben, etc.); excipients (mannitol, sorbitol, starch, gelatin, lactose, glucose, magnesium carbonate, calcium phosphate, etc.); thickeners (cetostearyl alcohol, dextrin, tragacanth, cellulose, hydroxyethylcellulose, hydroxypropylcellulose, etc.); stabilizers (ascorbic acid, aminoacetic acid, sodium alginate, sodium hydroxide, calcium stearate, etc.); moistening agents (benzalkonium chloride, glycerin, sorbitol, sodium docusate, propylene glycol, poloxamer, etc.); moistening adjustors (benzalkonium chloride, glycerin, sorbitol, sodium docusate, propylene glycol, poloxamer, etc.); and isotonicities (mannitol, etc.).

Although a suppository making use of an oily base as a principal base can be mentioned as one example of the preparation form of the rectal preparation according to this invention, the rectal preparation of this invention is not necessarily limited to a solid to semisolid preparation. For example, it is possible to formulate the preparation in the form of a rectal solution which uses a water-soluble polymer as a base, and to administer it by using a device similar to that employed for a usual enema. As a further alternative, it is also possible to fill the preparation of the present invention, said preparation being in the form of a solution, in capsules and to administer the capsules. As a still further alternative, it is also possible to fill a solution of the preparation of this invention in soft capsules (rectal capsules) and to administer the soft capsules.

The amount of the peptide to be incorporated in the rectal preparation varies, for example, depending on the kind of the peptide used. It is therefore necessary to add the peptide in its own effective amount. The effective amount can be determined in view of increases in its blood level by zoopery, clinical tests and/or the like. Where calcitonin is used as a peptide, for example, it is sufficient if calcitonin is incorporated in the preparation to give a calcitonin dose of 0.1-200 IU/kg or so.

Next, the present invention will be described in further detail by the following examples. It is however borne in mind that the present invention is by no means limited by the following examples.

### Example 1

Each rectal preparation of calcitonin was formulated by adding salmon calcitonin to a corresponding base for rectal preparations, said base having the composition described in a table set out below.

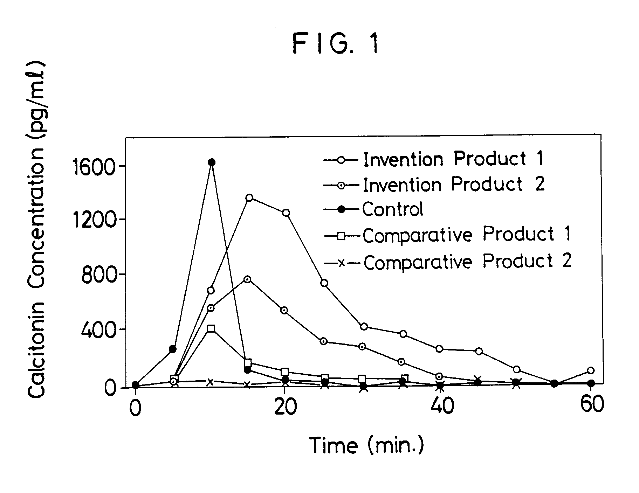

The preparation so obtained was administered rectally to a beagle (male, body weight: 10-12 kg, age: 11 months old), and time-dependent variations in the calcitonin level in serum were investigated. The test was carried out after the beagle had been fasted for 41 hours. Blood collection was effected in an amount of 2 mℓ per collection from a foreleg vein of the beagle immediately before the administration of the preparation and also at the 5th, 10th, 15th, 20th, 25th, 30th, 35th, 40th, 45th and 60th minutes after the administration. Each blood sample so obtained was centrifuged and the concentration of calcitonin in the serum was measured by radioimmunoassay (RIA). For the sake of reference, measurements were also made when 16.7 IU of salmon calcitonin were injected intramuscularly. The results are presented in FIGURE 1.

### (Compositions of bases)

| Ingredient | Base 1 | Base 2 | Base 3 |
|---|---|---|---|
| Pharmasol B-105 | 93.3 | 94.0 | 99.0 |
| Sodium caprate | 3.6 | 3.6 | 0 |
| Capric acid | 1.4 | 1.4 | 0 |
| Mannitol | 1.0 | 1.0 | 1.0 |
| Gabexate mesylate | 0.7 | 0 | 0 |

### (Compositions of rectal preparations)

- Invention Product 1:: Base 1 + salmon calcitonin (668 IU)
- Invention product 2:: Base 1 + salmon calcitonin (167 IU)
- Comparative Product 1:: Base 2 + salmon calcitonin (668 IU)
- Comparative Product 2:: Base 3 + salmon calcitonin (668 IU)
- Control:: Intramuscular injection of salmon calcitonin (16.7 IU)

As is apparent from FIGURE 1, the rectal compositions according to the present invention were superior in the transfer of calcitonin into serum compared with Comparative Product 1 in which the saturated fatty acid alone was used. The serum level of calcitonin increased only for a short period when administered by intramuscular injection, while the serum level of calcitonin remained high in the cases of the preparations according to the present invention. The preparations of the present invention are therefore expected to bring about excellent pharmacological efficacy.

### Example 2

Rectal preparations were formulated by adding 66.8 IU of salmon calcitonin to the respective rectal bases in Example 1.

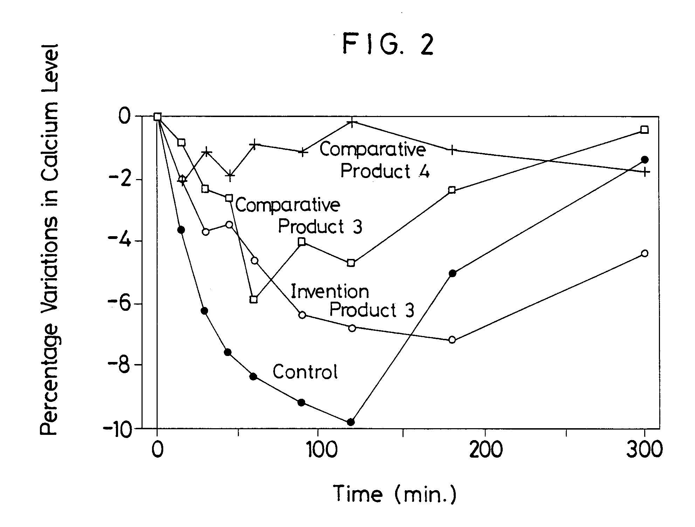

Each preparation was administered to the same beagle as that employed in Example 1, and blood collection was effected in an amount of 2 mℓ per collection from the foreleg vein of the beagle immediately before the administration of the preparation and also at the 15th, 30th, 45th, 60th, 90th, 120th, 180th and 300th minutes after the administration. After each blood sample so obtained was centrifuged and the concentration of calcium in the serum so obtained was measured by atomic absorption spectrometry, whereby variations in the serum level of calcium were investigated. The results are presented in FIGURE 2.

### (Compositions of rectal preparations)

- Invention Product 3:: Base 1 + salmon calcitonin (66.8 IU)
- Comparative Product 3:: Base 2 + salmon calcitonin (66.8 IU)
- Comparative product 3:: Base 2 + salmon calcitonin (66.8 IU)
- Comparative Product 4:: Base 3 + salmon calcitonin (66.8 IU)
- Control:: Intramuscular injection of salmon calcitonin (16.7 IU)

As is evident from the results in FIGURE 2, the saturated fatty acid and the protease inhibitor continuously lowered the serum level of calcium. Further, this action was superior to the comparative products which contained only the saturated fatty acid.

### Example 3

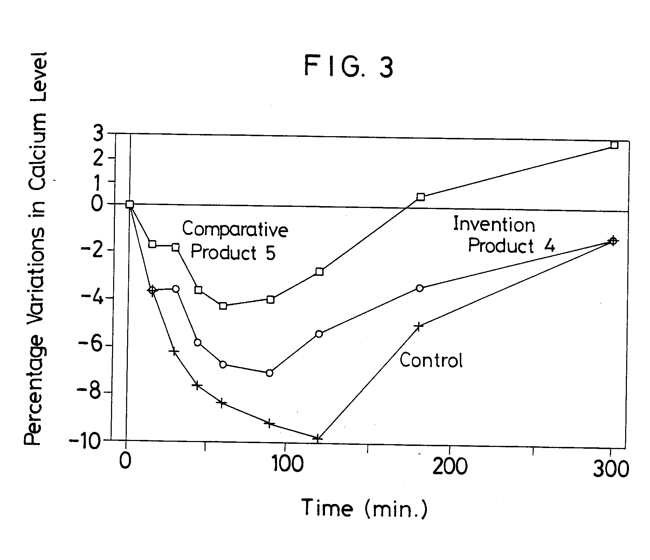

Rectal preparations were formulated in a similar manner to Example 2 except that the amount of salmon calcitonin was reduced to 16.7 IU. Each preparation was administered rectally to the beagle to investigate variations in the concentration of calcium in serum.
The results are presented in FIGURE 3.

### (Compositions of rectal preparations)

- Invention Product 4:: Base 1 + salmon calcitonin (16.7 IU)
- Comparative Product 5:: Base 2 + salmon calcitonin (16.7 IU)
- Control:: Intramuscular injection of salmon calcitonin (16.7 IU)

### Referential Example 1

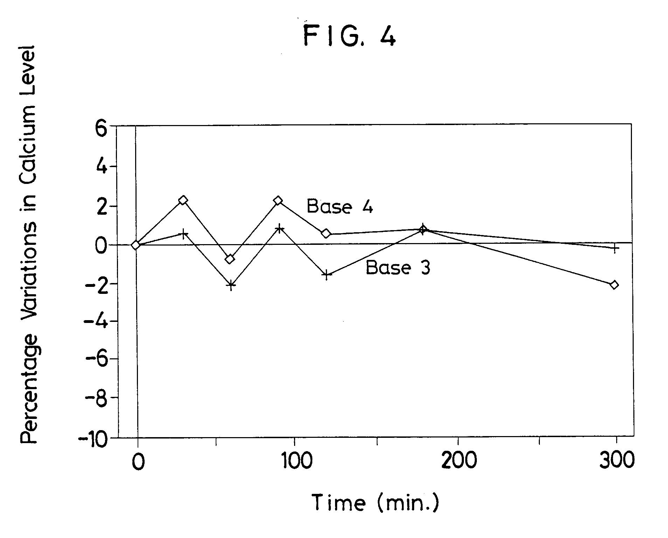

To demonstrate that the calcium concentration lowering action of each rectal preparation according to this invention is not the action of gabexate mesylate itself, a base for rectal preparations, said base being free of any special medicament (Base 1) and a similar base except for the incorporation of 0.7% of gabexate mesylate (Base 4) were prepared and, as in Example 2, those bases were separately administered to the beagle to investigate any influence to the blood level of calcium. The results are presented in FIGURE 4.

As is envisaged from FIGURE 4, the serum level of calcium did not vary substantially by the administration of gabexate mesylate alone.

### Industrial Applicability

According to the rectal preparation of this invention, the peptide whose absorption has heretofore been considered difficult can be efficiently absorbed through the rectal mucosa. Excellent pharmacological effects can therefore be obtained without relying upon injection which is accompanied by painful discomfort and also by restrictions to administration. In addition, the relatively expensive peptide can be formulated into a dosable preparation form while reducing its loss, so that the rectal preparation of this invention is also advantageous from the standpoint of economy.

## Claims

1. A rectal preparation of a low absorption peptide, comprising the low absorption peptide as an effective ingredient, a saturated fatty acid having 8-14 carbon atoms or a non-toxic salt thereof, and a protease inhibitor.

2. The rectal preparation of claim 1, wherein the proportion of the protease inhibitor is 0.1-5 wt.% based on the whole weight of the preparation.

3. The rectal preparation of claim 1, wherein the proportion of the saturated fatty acid having 8-14 carbon atoms or the non-toxic salt thereof is 0.1-20 wt.% based on the whole weight of the preparation.
